# EUROPEAN PATENT APPLICATION

(11) **EP 0 846 764 A2**
(43) Date of publication of application: **10.06.1998**
(21) Application number: 97309375.0
(22) Date of filing: 20.11.1997
(51) Int. Cl.: C12N 15/12, C07K 14/71, C07K 16/18, C12N 5/10, C12N 1/21, A61K 38/18, C12Q 1/68, G01N 33/53

(54) **Glial cell line-derived neurotrophic factor alpha receptor family**

(30) Priority: 27.11.1996 GB 9624677; 09.05.1997 GB 9709463
(71) Applicant: SMITHKLINE BEECHAM PLC, Brentford, Middlesex TW8 9EP (GB)
(72) Inventor: Lawrence, Geoffrey M.P., SmithKline Beecham Pharm., Harlow, Essex, CM19 5AW (GB)
(74) Representative: Connell, Anthony Christopher

(57) **Abstract**

GDNF alpha 3 receptor polypeptides and polynucleotides and methods for producing such polypeptides by recombinant techniques are disclosed. Also disclosed are methods for utilizing GDNF alpha 3 receptor polypeptides and polynucleotides in the design of protocols for the treatment of neurodegenerative diseases (such as Parkinson's Disease, amyotrophic lateral sclerosis (ALS), spinal muscular atrophy (SMA), Huntington's Disease, Alzheimer's Disease, diabetic neuropathy), muscular diseases (including the muscular dystrophies) and nerve and muscle trauma, among others and diagnostic assays for such conditions.

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to GDNF alpha receptor family, hereinafter referred to as GDNF alpha 3 receptor. The invention also relates to inhibiting or activating the action of such polynucleotides and polypeptides.

### BACKGROUND OF THE INVENTION

Glial cell line-derived neurotrophic factor (GDNF) is a 134 amino acid polypeptide that shows potent neurotrophic activity in a variety of central and peripheral neurons (L.-F H Lin et al., Science 260, 1130-1132, 1993). It has attracted attention as a potential therapy for Parkinson's Disease (PD) because of it's protective properties on dopaminergic neurons in the substantia nigra, the site of deterioration in PD. Studies on transgenic GDNF knock-outs in mice have confirmed the neurotrophic activity of GDNF but also revealed a central role for this molecule in kidney formation and the innervation of the enteric system (M. Sanchez et al., Nature 382, 70-73, 1996; J. Pichel et al., Nature 382, 73-76, 1996; M. Moore et al., Nature 382, 76-79, 1996). The receptor complex for GDNF has been identified by a number of groups and shown to comprise the orphan tyrosine kinase receptor Ret (M.Trupp et al., Nature 381, 785-789, 1996; P. Durbec et al., Nature 381, 789-793, 1996) and an ancilliary component, GDNF-alpha (J. Treanor et al., Nature 382, 80-83, 1996 and S. Jing et al., Cell 85 1113-1124, 1996). It is believed an ancillary binding part of the receptor complex, GDNF alpha receptor, modulates GDNF stimulated signalling of the Ret receptor, which may be essential since Ret is a proto-oncogene. More recently, a GDNF related polypeptide called Neurturin has been identified (P. Kotzbauer et al., Nature 384, 467-470, 1996). It's receptor components however remain unknown. This raises the possibility of further alpha receptors being identified and possibly additional Ret-like receptors. In addition other GDNF-like homologues may yet be identified, thereby forming a family of trophic polypeptides and receptor complexes.

### SUMMARY OF THE INVENTION

In one aspect, the invention relates to GDNF alpha 3 receptor polypeptides and recombinant materials and methods for their production. Another aspect of the invention relates to methods for using such GDNF alpha 3 receptor polypeptides and polynucleotides. Such uses include the treatment of neurodegenerative diseases (such as Parkinson's Disease, amyotrophic lateral sclerosis (ALS), spinal muscular atrophy (SMA), Huntington's Disease, Alzheimer's Disease, diabetic neuropathy), muscular diseases (including the muscular dystrophies) and nerve and muscle trauma, among others. In still another aspect, the invention relates to methods to identify agonists and antagonists using the materials provided by the invention, and treating conditions associated with GDNF alpha 3 receptor imbalance with the identified compounds. Yet another aspect of the invention relates to diagnostic assays for detecting diseases associated with inappropriate GDNF alpha 3 receptor activity or levels.

### DESCRIPTION OF THE INVENTION

### Polypeptides of the Invention

In one aspect, the present invention relates to GDNF alpha 3 receptor polypeptides. The GDNF alpha 3 receptor polypeptides include the polypeptides of SEQ ID NO:2; as well as polypeptides comprising the amino acid sequence of SEQ ID NO:2; and polypeptides comprising the amino acid sequence which have at least 80% identity to that of SEQ ID NO:2 over the entire length of SEQ ID NO:2, and still more preferably at least 90% identity, and even still more preferably at least 95% identity to SEQ ID NO: 2. Furthermore, those with at least 97-99% are highly preferred. Also included within GDNF alpha 3 receptor polypeptides are polypeptides having the amino acid sequence which have at least 80% identity to the polypeptide having the amino acid sequence of SEQ ID NO: 2 over its entire length, and still more preferably at least 90% identity, and even still more preferably at least 95% identity to SEQ ID NO: 2. Furthermore, those with at least 97-99% are highly preferred. Preferably GDNF alpha 3 receptor polypeptides exhibit at least one biological activity of the receptor.

The GDNF alpha 3 receptor polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the GDNF alpha 3 receptor polypeptides are also included in the invention. A fragment is a polypeptide having an amino acid sequence that entirely is the same as part, but not all, of the amino acid sequence of the aforementioned GDNF alpha 3 receptor polypeptides. As with GDNF alpha 3 receptor polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of GDNF alpha 3 receptor polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of GDNF alpha 3 receptor polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate receptor activity, including those with a similar activity or an improved activity, or with a decreased undesirable activity. Also included are those that are antigenic or immunogenic in an animal, especially in a human.

Preferably, all of these polypeptide fragments retain the biological activity of the receptor, including antigenic activity.

Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The GDNF alpha 3 receptor polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to GDNF alpha 3 receptor polynucleotides. GDNF alpha 3 receptor polynucleotides include isolated polynucleotides which encode the GDNF alpha 3 receptor polypeptides and fragments, and polynucleotides closely related thereto. More specifically, GDNF alpha 3 receptor polynucleotide of the invention include a polynucleotide comprising the nucleotide sequence set forth in SEQ ID NO: 1 encoding a GDNF alpha 3 receptor polypeptide of SEQ ID NO: 2, and polynucleotides having the particular sequences of SEQ ID NO:1. GDNF alpha 3 receptor polynucleotides further include a polynucleotide comprising a nucleotide sequence that has at least 80% identity to a nucleotide sequence encoding the GDNF alpha 3 receptor polypeptide of SEQ ID NO:2 over the entire coding region, and a polynucleotide that is at least 80% identical to that having SEQ ID NO: 1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under GDNF alpha 3 receptor polynucleotides are a nucleotide sequence which has sufficient identity to a nucleotide sequence contained in SEQ ID NO: 1 to hybridize under conditions useable for amplification or for use as a probe or marker. The invention also provides polynucleotides which are complementary to such GDNF alpha 3 receptor polynucleotides.

GDNF alpha 3 receptor of the invention is structurally related to other proteins of the GDNF alpha receptor family, as shown by the results of sequencing the cDNA encoding human GDNF alpha 3 receptor. The cDNA sequence of SEQ ID NO:1 contains an open reading frame (nucleotide number 1 to 1200) encoding a polypeptide of 400 amino acids of SEQ ID NO:2. The amino acid sequence of SEQ ID NO:2 has about 36% identity (using Bestfit) in 400 amino acid residues with GDNF alpha receptor (J. Treanor et al., Nature, 382, 80-83, 1996; S. Jing et al., Cell, 85, 1113-1124, 1996). The nucleotide sequence of SEQ ID NO: 1 has about 61% identity (using Bestfit) in 490 nucleotide residues with rat GDNF alpha receptor (J. Treanor et al., Nature, 382, 80-83, 1996; S. Jing et al., Cell, 85, 1113-1124, 1996).

The GDNF alpha 3 receptor has a signal sequence (for directing the receptor through the secretary pathway) with a putative cleavage site between the alanine residues at positions 29 and 30. In addition there is a hydrophobic region at the C-terminus of the receptor which could function in tethering the receptor to the outside of the membrane via a glycosyl-phosphatidyl-inositol (GPI) anchor, a characteristic of the GDNF alpha receptor family. Accordingly, in a further aspect, a preferred fragment is the amino acid sequence of SEQ ID NO:2, from amino acid 30 (A) to amino acid 400 (W).

The programme of work leading to the determination of SEQ ID NO:1 to 4 started with the preliminary identification of shorter length gene fragments (ESTs). Accordingly, in a further aspect, the present invention provides for a GDNF alpha 3 receptor characterised by the deduced amino acid sequence of SEQ ID NO 6; or a fragment thereof, and for polynucleotides which encode such polypeptides, in particular, the polynucleotide comprising the partial DNA sequence given in SEQ ID NO 5.

The DNA sequence given in SEQ ID NO:5 is a partial sequence, which represents some 30-50% of the coding region of the full-length clone. It will be appreciated that the deduced amino acid sequence given in SEQ ID NO:6 is thus also a partial sequence, representing some 30-50% of the complete amino acid sequence. The partial DNA and amino acid sequences are sufficient to characterise the corresponding full length sequences. Comparison of the partial and full length sequences shows that the partial sequence is incomplete at the 5' end. The skilled artisan will readily appreciate that the polynucleotide and polypeptide of SEQ ID NO:5 and 6 respectively are fragments of the the polynucleotides and polypeptides of SEQ ID NO: 1 and 2, respectively. In references herein to SEQ ID NO:1, SEQ ID NO:1 may be replaced by SEQ ID NO:3. . In references herein to SEQ ID NO:2, SEQ ID NO:2 may be replaced by SEQ ID NO:4.

SEQ ID NO:1 differs from SEQ ID NO:3 in one base (C rather A at position 176). SEQ ID NO:3 was the first identified full length sequence. This was subsequently amended to SEQ ID NO:1 as a consequence of further confirmatory sequencing. A consequence of this is the change in amino acid 59 from D to A between corresponding deduced amino acid sequences SEQ ID NO:2 and SEQ ID NO:4.

One polynucleotide of the present invention encoding GDNF alpha 3 receptor may be obtained using standard cloning and screening, from a cDNA library derived from mRNA in cells of human testes, fetal heart and skeletal muscle using the expressed sequence tag (EST) analysis (Adams, M.D., *et al. Science* (1991) 252:1651-1656; Adams, M.D. *et al., Nature,* (1992) 355:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding GDNF alpha 3 receptor polypeptide of SEQ ID NO:2 may be identical to the polypeptide encoding sequence hereinbefore described (nucleotide number 1 to 1200 of SEQ ID NO:1), or it may be a different sequence, which as a result of the redundancy (degeneracy) of the genetic code, also encodes the polypeptide of SEQ ID NO:2.

When the polynucleotides of the invention are used for the recombinant production of GDNF alpha 3 receptor polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain noncoding 5' and 3' sequences, such as transcribed, non-translated sequences, splicing and polyadenylation signals, ribosome binding sites and sequences that stabilize mRNA.

Further preferred embodiments are polynucleotides encoding GDNF alpha 3 receptor variants comprising the amino acid sequence of GDNF alpha 3 receptor polypeptide of SEQ ID NO:2 in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, may be used as hybridization probes for cDNA and genomic DNA, to isolate full-length cDNAs and genomic clones encoding GDNF alpha 3 receptor and to isolate cDNA and genomic clones of other genes that have a high sequence similarity to the GDNF alpha 3 receptor gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding GDNF alpha 3 receptor polypeptide comprises the steps of screening an appropriate library under stingent hybridization conditions with a labeled probe having the sequence of SEQ ID NO: 1 or a fragment thereof (including that of SEQ ID NO: 5), and isolating full-length cDNA and genomic clones containing said polynucleotide sequence. Such hybridization techniques are well known to those of skill in the art. Thus in another aspect, GDNF alpha 3 receptor polynucleotides of the present invention further include a nucleotide sequence comprising a nucleotide sequence that hybridize under stringent condition to a nucleotide sequence having SEQ ID NO: 1 or a fragment thereof. Also included with GDNF alpha 3 receptor polypeptides are polypeptide comprising amino acid sequence encoded by nucleotide sequence obtained by the above hybridization condition. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. Coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the GDNF alpha 3 receptor polypeptide is to be expressed for use in screening assays, generally, it is preferred that the polypeptide be produced at the surface of the cell. In this event, the cells may be harvested prior to use in the screening assay. If GDNF alpha 3 receptor polypeptide is secreted into the medium, the medium can be recovered in order to recover and purify the polypeptide; if produced intracellularly, the cells must first be lysed before the polypeptide is recovered.

GDNF alpha 3 receptor polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of GDNF alpha 3 receptor polynucleotides for use as diagnostic reagents. Detection of a mutated form of GDNF alpha 3 receptor gene associated with a dysfunction will provide a diagnostic tool that can add to or define a diagnosis of a disease or susceptibility to a disease which results from under-expression, over-expression or altered expression of GDNF alpha 3 receptor. Individuals carrying mutations in the GDNF alpha 3 receptor gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled GDNF alpha 3 receptor nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising GDNF alpha 3 receptor nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to neurodegenerative diseases (such as Parkinson's Disease, amyotrophic lateral sclerosis (ALS), spinal muscular atrophy (SMA), Huntington's Disease, Alzheimer's Disease, diabetic neuropathy) and muscular diseases (including the muscular dystrophies) through detection of mutation in the GDNF alpha 3 receptor gene by the methods described.

In addition, neurodegenerative diseases (such as Parkinson's Disease, amyotrophic lateral sclerosis (ALS), spinal muscular atrophy (SMA), Huntington's Disease, Alzheimer's Disease, diabetic neuropathy) and muscular diseases (including the muscular dystrophies), can be diagnosed by methods comprising determining from a sample derived from a subject an abnormally decreased or increased level of GDNF alpha 3 receptor polypeptide or GDNF alpha 3 receptor mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an GDNF alpha 3 receptor, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined. If a mutation is observed in some or all of the affected individuals but not in any normal individuals, then the mutation is likely to be the causative agent of the disease.

The novel GDNF alpha 3 receptor has been mapped to chromosome 5q31 by radiation hybrid mapping. Several disease loci map to this position including Limb-girdle muscular dystrophy type 1A, autosomal nonsyndromic sensoneural deafness and corneal dystrophy, granular type.

### Antibodies

The polypeptides of the invention or their fragments or analogs thereof, or cells expressing them can also be used as immunogens to produce antibodies immunospecific for the GDNF alpha 3 receptor polypeptides. The term "immunospecific" means that the antibodies have substantiall greater affinity for the polypeptides of the invention than their affinity for other related polypeptides in the prior art.

Antibodies generated against the GDNF alpha 3 receptor polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983)4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against GDNF alpha 3 receptor polypeptides may also be employed to treat neurodegenerative diseases (such as Parkinson's Disease, amyotrophic lateral sclerosis (ALS), spinal muscular atrophy (SMA), Huntington's Disease, Alzheimer's Disease, diabetic neuropathy), muscular diseases (including the muscular dystrophies) and nerve and muscle trauma, among others.

### Vaccines

Another aspect of the invention relates to a method for inducing an immunological response in a mammal which comprises inoculating the mammal with GDNF alpha 3 receptor polypeptide, or a fragment thereof, adequate to produce antibody and/or T cell immune response to protect said animal from neurodegenerative diseases (such as Parkinson's Disease, amyotrophic lateral sclerosis (ALS), spinal muscular atrophy (SMA), Huntington's Disease, Alzheimer's Disease, diabetic neuropathy), muscular diseases (including the muscular dystrophies) and nerve and muscle trauma, among others. Yet another aspect of the invention relates to a method of inducing immunological response in a mammal which comprises, delivering GDNF alpha 3 receptor polypeptide via a vector directing expression of GDNF alpha 3 receptor polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a GDNF alpha 3 receptor polypeptide wherein the composition comprises a GDNF alpha 3 receptor polypeptide or GDNF alpha 3 receptor gene. The vaccine formulation may further comprise a suitable carrier. Since GDNF alpha 3 receptor polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The GDNF alpha 3 receptor polypeptide of the present invention may be employed in a screening process for compounds which bind the receptor and which activate (agonists) or inhibit activation of (antagonists) the receptor polypeptide of the present invention. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al., Current Protocols in Immunology* 1(2):Chapter 5 (1991).

GDNF alpha 3 receptor polypeptides are responsible for many biological functions, including many pathologies. Accordingly, it is desirous to find compounds and drugs which stimulate GDNF alpha 3 receptor on the one hand and which can inhibit the function of GDNF alpha 3 receptor on the other hand. In general, agonists are employed for therapeutic and prophylactic purposes for such conditions as neurodegenerative diseases (such as Parkinson's Disease, amyotrophic lateral sclerosis (ALS), spinal muscular atrophy (SMA), Huntington's Disease, Alzheimer's Disease, diabetic neuropathy), muscular diseases (including the muscular dystrophies) and nerve and muscle trauma. Antagonists may be employed for a variety of therapeutic and prophylactic purposes for such conditions as neurodegenerative diseases (such as Parkinson's Disease, amyotrophic lateral sclerosis (ALS), spinal muscular atrophy (SMA), Huntington's Disease, Alzheimer's Disease, diabetic neuropathy), muscular diseases (including the muscular dystrophies) and nerve and muscle trauma.

In general, such screening procedures involve producing appropriate cells which express the receptor polypeptide of the present invention on the surface thereof. Such cells include cells from mammals, yeast, *Drosophila* or *E. coli.* Cells expressing the receptor (or cell membrane containing the expressed receptor) are then contacted with a test compound to observe binding, or stimulation or inhibition of a functional response.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed. Standard methods for conducting such screening assays are well understood in the art.

Examples of potential GDNF alpha 3 receptor antagonists include antibodies or, in some cases, oligonucleotides or proteins which are closely related to the ligand of the GDNF alpha 3 receptor, e.g., a fragment of the ligand, or small molecules which bind to the receptor but do not elicit a response, so that the activity of the receptor is prevented.

### Prophylactic and Therapeutic Methods

This invention provides methods of treating abnormal conditions such as, neurodegenerative diseases (such as Parkinson's Disease, amyotrophic lateral sclerosis (ALS), spinal muscular atrophy (SMA), Huntington's Disease, Alzheimer's Disease, diabetic neuropathy), muscular diseases (including the muscular dystrophies) and nerve and muscle trauma, related to both an excess of and insufficient amounts of GDNF alpha 3 receptor activity.

If the activity of GDNF alpha 3 receptor is in excess, several approaches are available. One approach comprises administering to a subject an inhibitor compound (antagonist) as hereinabove described along with a pharmaceutically acceptable carrier in an amount effective to inhibit activation by blocking binding of ligands to the GDNF alpha 3 receptor, or by inhibiting a second signal, and thereby alleviating the abnormal condition. In another approach, soluble forms of GDNF alpha 3 receptor polypeptides still capable of binding the ligand in competition with endogenous GDNF alpha 3 receptor may be administered. Typical embodiments of such competitors comprise fragments of the GDNF alpha 3 receptor polypeptide.

In still another approach, expression of the gene encoding endogenous GDNF alpha 3 receptor can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

For treating abnormal conditions related to an under-expression of GDNF alpha 3 receptor and its activity, several approaches are also available. One approach comprises administering to a subject a therapeutically effective amount of a compound which activates GDNF alpha 3 receptor, i.e., an agonist as described above, in combination with a pharmaceutically acceptable carrier, to thereby alleviate the abnormal condition. Alternatively, gene therapy may be employed to effect the endogenous production of GDNF alpha 3 receptor by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of GDNF alpha 3 receptor polypeptides in combination with a suitable pharmaceutical carrier.

### Formulation and Administration

Peptides, such as the soluble form of GDNF alpha 3 receptor polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

### Definitions

The following definitions are provided to facilitate understanding of certain terms used frequently hereinbefore.

"GDNF alpha 3 receptor" refers, among others, to a polypeptide comprising the amino acid sequence set forth in SEQ ID NO:2, or an allelic variant thereof.

"Receptor Activity" or "Biological Activity of the Receptor" refers to the metabolic or physiologic function of said GDNF alpha 3 receptor including similar activities or improved activities or these activities with decreased undesirable side-effects. Also included are antigenic and immunogenic activities of said GDNF alpha 3 receptor.

"GDNF alpha 3 receptor gene" refers to a polynucleotide comprising the nucleotide sequence set forth in SEQ ID NO: 1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663 :48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Examples

The examples below are carried out using standard techniques, which are well known and routine to those of skill in the art, except where otherwise described in detail. The examples illustrate, but do not limit the invention.

### Example 1

The full-length sequence was determined by a combination of 5' RACE (rapid amplification of cDNA ends) using Marathon-ready^{™} testes cDNA (Clontech Laboratories Inc.) and the identification of overlapping ESTs.

### Example 2

The full-length sequence is determined by isolating and sequencing genomic fragments encoding specific fragments of the GDNF alpha 3 receptor gene using the Genome Walker^{™} kit (Clontech Laboratories Inc.). The specific genomic fragments are generated by PCR (polymerase chain reaction) using gene specific primers in combination with fixed 'adapter' primers which bind to synthetic sequences ligated to the ends of fragmented genomic DNA.

SEQUENCE LISTING

(1) GENERAL INFORMATION

(i) APPLICANT
(A) NAME: SmithKline Beecham plc
(B) STREET: New Horizons Court
(C) CITY: Brentford
(D) STATE OR PROVINCE: Middlesex
(E) COUNTRY: Great Britain
(F) POSTAL CODE: TW8 9EP

(ii) TITLE OF THE INVENTION: Novel Compounds

(iii) NUMBER OF SEQUENCES: 6

(iv) COMPUTER-READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(v) CURRENT APPLICATION DATA:
(A) APPLICATION NUMBER:

(2) INFORMATION FOR SEQ ID NO:1:

(i) SEQUENCE CHARACTERISTICS:
(A) LENGTH: 1200 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

ATGGTGCGCC CCCTGAACCC GCGACCGCTG CCGCCCGTAG TCCTGATGTT GCTGCTGCTG 60
CTGCCGCCGT CGCCGCTGCC TCTCGCAGCC GGAGACCCCC TTCCCACAGA AAGCCGACTC 120
ATGAACAGCT GTCTCCAGGC CAGGAGGAAG TGCCAGGCTG ATCCCACCTG CAGTGCTGCC 180
TACCACCACC TGGATTCCTG CACCTCTAGC ATAAGCACCC CACTGCCCTC AGAGGAGCCT 240

TCGGTCCCTG CTGACTGCCT GGAGGCAGCA CAGCAACTCA GGAACAGCTC TCTGATAGGC 300
TGCATGTGCC ACCGGCGCAT GAAGAACCAG GTTGCCTGCT TGGACATCTA TTGGACCGTT 360
CACCGTGCCC GCAGCCTTGG TAACTATGAG CTGGATGTCT CCCCCTATGA AGACACAGTG 420
ACCAGCAAAC CCTGGAAAAT GAATCTCAGC AAACTGAACA TGCTCAAACC AGACTCAGAC 480
CTCTGCCTCA AGTTTGCCAT GCTGTGTACT CTCAATGACA AGTGTGACCG GCTGCGCAAG 540
GCCTACGGGG AGGCGTGCTC CGGGCCCCAC TGCCAGCGCC ACGTCTGCCT CAGGCAGCTG 600
CTCACTTTCT TCGAGAAGGC CGCCGAGCCC CACGCGCAGG GCCTGCTACT GTGCCCATGT 660
GCCCCCAACG ACCGGGGCTG CGGGGAGCGC CGGCGCAACA CCATCGCCCC CAACTGCGCG 720
CTGCCGCCTG TGGCCCCCAA CTGCCTGGAG CTGCGGCGCC TCTGCTTCTC CGACCCGCTT 780
TGCAGATCAC GCCTGGTGGA TTTCCAGACC CACTGCCATC CCATGGACAT CCTAGGAACT 840
TGTGCAACAG AGCAGTCCAG ATGTCTACGA GCATACCTGG GGCTGATTGG GACTGCCATG 900
ACCCCCAACT TTGTCAGCAA TGTCAACACC AGTGTTGCCT TAAGCTGCAC CTGCCGAGGC 960
AGTGGCAACC TGCAGGAGGA GTGTGAAATG CTGGAAGGGT TCTTCTCCCA CAACCCCTGC 1020
CTCACGGAGG CCATTGCAGC TAAGATGCGT TTTCACAGCC AACTCTTCTC CCAGGACTGG 1080
CCACACCCTA CCTTTGCTGT GATGGCACAC CAGAATGAAA ACCCTGCTGT GAGGCCACAG 1140
CCCTGGGTGC CCTCTCTTTT CTCCTGCACG CTTCCCTTGA TTCTGCTCCT GAGCCTATGG 1200

(2) INFORMATION FOR SEQ ID NO:2:

(i) SEQUENCE CHARACTERISTICS:
(A) LENGTH: 400 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:2:

Met Val Arg Pro Leu Asn Pro Arg Pro Leu Pro Pro Val Val Leu Met
1               5                   10                  15
Leu Leu Leu Leu Leu Pro Pro Ser Pro Leu Pro Leu Ala Ala Gly Asp
            20                  25                  30
Pro Leu Pro Thr Glu Ser Arg Leu Met Asn Ser Cys Leu Gln Ala Arg
        35                  40                  45
Arg Lys Cys Gln Ala Asp Pro Thr Cys Ser Ala Ala Tyr His His Leu
    50                  55                  60
Asp Ser Cys Thr Ser Ser Ile Ser Thr Pro Leu Pro Ser Glu Glu Pro
65                  70                  75                  80
Ser Val Pro Ala Asp Cys Leu Glu Ala Ala Gln Gln Leu Arg Asn Ser
                85                  90                  95
Ser Leu Ile Gly Cys Met Cys His Arg Arg Met Lys Asn Gln Val Ala

            100                 105                 110
Cys Leu Asp Ile Tyr Trp Thr Val His Arg Ala Arg Ser Leu Gly Asn
        115                 120                 125
Tyr Glu Leu Asp Val Ser Pro Tyr Glu Asp Thr Val Thr Ser Lys Pro
    130                 135                 140
Trp Lys Met Asn Leu Ser Lys Leu Asn Met Leu Lys Pro Asp Ser Asp
145                 150                 155                 160
Leu Cys Leu Lys Phe Ala Met Leu Cys Thr Leu Asn Asp Lys Cys Asp
                165                 170                 175
Arg Leu Arg Lys Ala Tyr Gly Glu Ala Cys Ser Gly Pro His Cys Gln
            180                 185                 190
Arg His Val Cys Leu Arg Gln Leu Leu Thr Phe Phe Glu Lys Ala Ala
        195                 200                 205
Glu Pro His Ala Gln Gly Leu Leu Leu Cys Pro Cys Ala Pro Asn Asp
    210                 215                 220
Arg Gly Cys Gly Glu Arg Arg Arg Asn Thr Ile Ala Pro Asn Cys Ala
225                 230                 235                 240
Leu Pro Pro Val Ala Pro Asn Cys Leu Glu Leu Arg Arg Leu Cys Phe
                245                 250                 255
Ser Asp Pro Leu Cys Arg Ser Arg Leu Val Asp Phe Gln Thr His Cys
            260                 265                 270
His Pro Met Asp Ile Leu Gly Thr Cys Ala Thr Glu Gln Ser Arg Cys
        275                 280                 285
Leu Arg Ala Tyr Leu Gly Leu Ile Gly Thr Ala Met Thr Pro Asn Phe
    290                 295                 300
Val Ser Asn Val Asn Thr Ser Val Ala Leu Ser Cys Thr Cys Arg Gly
305                 310                 315                 320
Ser Gly Asn Leu Gln Glu Glu Cys Glu Met Leu Glu Gly Phe Phe Ser
                325                 330                 335
His Asn Pro Cys Leu Thr Glu Ala Ile Ala Ala Lys Met Arg Phe His
            340                 345                 350
Ser Gln Leu Phe Ser Gln Asp Trp Pro His Pro Thr Phe Ala Val Met
        355                 360                 365
Ala His Gln Asn Glu Asn Pro Ala Val Arg Pro Gln Pro Trp Val Pro
    370                 375                 380
Ser Leu Phe Ser Cys Thr Leu Pro Leu Ile Leu Leu Leu Ser Leu Trp
385                 390                 395                 400

(2) INFORMATION FOR SEQ ID NO:3:

(i) SEQUENCE CHARACTERISTICS:
(A) LENGTH: 1200 base pairs

(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

ATGGTGCGCC CCCTGAACCC GCGACCGCTG CCGCCCGTAG TCCTGATGTT GCTGCTGCTG 60
CTGCCGCCGT CGCCGCTGCC TCTCGCAGCC GGAGACCCCC TTCCCACAGA AAGCCGACTC 120
ATGAACAGCT GTCTCCAGGC CAGGAGGAAG TGCCAGGCTG ATCCCACCTG CAGTGATGCC 180
TACCACCACC TGGATTCCTG CACCTCTAGC ATAAGCACCC CACTGCCCTC AGAGGAGCCT 240
TCGGTCCCTG CTGACTGCCT GGAGGCAGCA CAGCAACTCA GGAACAGCTC TCTGATAGGC 300
TGCATGTGCC ACCGGCGCAT GAAGAACCAG GTTGCCTGCT TGGACATCTA TTGGACCGTT 360
CACCGTGCCC GCAGCCTTGG TAACTATGAG CTGGATGTCT CCCCCTATGA AGACACAGTG 420
ACCAGCAAAC CCTGGAAAAT GAATCTCAGC AAACTGAACA TGCTCAAACC AGACTCAGAC 480
CTCTGCCTCA AGTTTGCCAT GCTGTGTACT CTCAATGACA AGTGTGACCG GCTGCGCAAG 540
GCCTACGGGG AGGCGTGCTC CGGGCCCCAC TGCCAGCGCC ACGTCTGCCT CAGGCAGCTG 600
CTCACTTTCT TCGAGAAGGC CGCCGAGCCC CACGCGCAGG GCCTGCTACT GTGCCCATGT 660
GCCCCCAACG ACCGGGGCTG CGGGGAGCGC CGGCGCAACA CCATCGCCCC CAACTGCGCG 720
CTGCCGCCTG TGGCCCCCAA CTGCCTGGAG CTGCGGCGCC TCTGCTTCTC CGACCCGCTT 780
TGCAGATCAC GCCTGGTGGA TTTCCAGACC CACTGCCATC CCATGGACAT CCTAGGAACT 840
TGTGCAACAG AGCAGTCCAG ATGTCTACGA GCATACCTGG GGCTGATTGG GACTGCCATG 900
ACCCCCAACT TTGTCAGCAA TGTCAACACC AGTGTTGCCT TAAGCTGCAC CTGCCGAGGC 960
AGTGGCAACC TGCAGGAGGA GTGTGAAATG CTGGAAGGGT TCTTCTCCCA CAACCCCTGC 1020
CTCACGGAGG CCATTGCAGC TAAGATGCGT TTTCACAGCC AACTCTTCTC CCAGGACTGG 1080
CCACACCCTA CCTTTGCTGT GATGGCACAC CAGAATGAAA ACCCTGCTGT GAGGCCACAG 1140
CCCTGGGTGC CCTCTCTTTT CTCCTGCACG CTTCCCTTGA TTCTGCTCCT GAGCCTATGG 1200

(2) INFORMATION FOR SEQ ID NO:4:

(i) SEQUENCE CHARACTERISTICS:
(A) LENGTH: 400 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:4:

Met Val Arg Pro Leu Asn Pro Arg Pro Leu Pro Pro Val Val Leu Met

1                   5                   10                  15
Leu Leu Leu Leu Leu Pro Pro Ser Pro Leu Pro Leu Ala Ala Gly Asp
                20                  25                  30
Pro Leu Pro Thr Glu Ser Arg Leu Met Asn Ser Cys Leu Gln Ala Arg
            35                  40                  45
Arg Lys Cys Gln Ala Asp Pro Thr Cys Ser Asp Ala Tyr His His Leu
    50                  55                  60
Asp Ser Cys Thr Ser Ser Ile Ser Thr Pro Leu Pro Ser Glu Glu Pro
65                  70                  75                  80
Ser Val Pro Ala Asp Cys Leu Glu Ala Ala Gln Gln Leu Arg Asn Ser
                85                  90                  95
Ser Leu Ile Gly Cys Met Cys His Arg Arg Met Lys Asn Gln Val Ala
                100                 105                 110
Cys Leu Asp Ile Tyr Trp Thr Val His Arg Ala Arg Ser Leu Gly Asn
            115                 120                 125
Tyr Glu Leu Asp Val Ser Pro Tyr Glu Asp Thr Val Thr Ser Lys Pro
    130                 135                 140
Trp Lys Met Asn Leu Ser Lys Leu Asn Met Leu Lys Pro Asp Ser Asp
145                 150                 155                 160
Leu Cys Leu Lys Phe Ala Met Leu Cys Thr Leu Asn Asp Lys Cys Asp
                165                 170                 175
Arg Leu Arg Lys Ala Tyr Gly Glu Ala Cys Ser Gly Pro His Cys Gln
                180                 185                 190
Arg His Val Cys Leu Arg Gln Leu Leu Thr Phe Phe Glu Lys Ala Ala
            195                 200                 205
Glu Pro His Ala Gln Gly Leu Leu Leu Cys Pro Cys Ala Pro Asn Asp
    210                 215                 220
Arg Gly Cys Gly Glu Arg Arg Arg Asn Thr Ile Ala Pro Asn Cys Ala
225                 230                 235                 240
Leu Pro Pro Val Ala Pro Asn Cys Leu Glu Leu Arg Arg Leu Cys Phe
                245                 250                 255
Ser Asp Pro Leu Cys Arg Ser Arg Leu Val Asp Phe Gln Thr His Cys
                260                 265                 270
His Pro Met Asp Ile Leu Gly Thr Cys Ala Thr Glu Gln Ser Arg Cys
            275                 280                 285
Leu Arg Ala Tyr Leu Gly Leu Ile Gly Thr Ala Met Thr Pro Asn Phe
    290                 295                 300
Val Ser Asn Val Asn Thr Ser Val Ala Leu Ser Cys Thr Cys Arg Gly
305                 310                 315                 320
Ser Gly Asn Leu Gln Glu Glu Cys Glu Met Leu Glu Gly Phe Phe Ser
                325                 330                 335
His Asn Pro Cys Leu Thr Glu Ala Ile Ala Ala Lys Met Arg Phe His

            340                 345                 350
Ser Gln Leu Phe Ser Gln Asp Trp Pro His Pro Thr Phe Ala Val Met
        355                 360                 365
Ala His Gln Asn Glu Asn Pro Ala Val Arg Pro Gln Pro Trp Val Pro
    370                 375                 380
Ser Leu Phe Ser Cys Thr Leu Pro Leu Ile Leu Leu Leu Ser Leu Trp
385                 390                 395                 400

(2) INFORMATION FOR SEQ ID NO:5:

(i) SEQUENCE CHARACTERISTICS:
(A) LENGTH: 519 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

GAGCGCCGGC GCAACACCAT CGCCCCCAAC TGCGCGCTGC CGCCTGTGGC CCCCAACTGC 60
CTGGAGCTGC GGCGCCTCTG CTTCTCCGAC CCGCTTTGCA GATCACGCCT GGTGGATTTC 120
CAGACCCACT GCCATCCCAT GGACATCCTA GGAACTTGTG CAACAGAGCA GTCCAGATGT 180
CTACGAGCAT ACCTGGGGCT GATTGGGACT GCCATGACCC CCAACTTTGT CAGCAATGTC 240
AACACCAGTG TTGCCTTAAG CTGCACCTGC CGAGGCAGTG GCAACCTGCA GGAGGAGTGT 300
GAAATGCTGG AAGGGTTCTT CTCCCACAAC CCCTGCCTCA CGGAGGCCAT TGCAGCTAAG 360
ATGCGTTTTC ACAGCCAACT CTTCTCCCAG GACTGGCCAC ACCCTACCTT TGCTGTGATG 420
GCACACCAGA ATGAAAACCC TGCTGTGAGG CCACAGCCCT GGGTGCCCTC TCTTTTCTCC 480
TGCACGCTTC CCTTGATTCT GCTCCTGAGC CTATGGTAG 519

(2) INFORMATION FOR SEQ ID NO:6:

(i) SEQUENCE CHARACTERISTICS:
(A) LENGTH: 172 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:6:

Glu Arg Arg Arg Asn Thr Ile Ala Pro Asn Cys Ala Leu Pro Pro Val
1               5                   10                  15
Ala Pro Asn Cys Leu Glu Leu Arg Arg Leu Cys Phe Ser Asp Pro Leu
            20                  25                  30
Cys Arg Ser Arg Leu Val Asp Phe Gln Thr His Cys His Pro Met Asp
        35                  40                  45
Ile Leu Gly Thr Cys Ala Thr Glu Gln Ser Arg Cys Leu Arg Ala Tyr
    50                  55                  60
Leu Gly Leu Ile Gly Thr Ala Met Thr Pro Asn Phe Val Ser Asn Val
65                  70                  75                  80
Asn Thr Ser Val Ala Leu Ser Cys Thr Cys Arg Gly Ser Gly Asn Leu
                85                  90                  95
Gln Glu Glu Cys Glu Met Leu Glu Gly Phe Phe Ser His Asn Pro Cys
            100                 105                 110
Leu Thr Glu Ala Ile Ala Ala Lys Met Arg Phe His Ser Gln Leu Phe
        115                 120                 125
Ser Gln Asp Trp Pro His Pro Thr Phe Ala Val Met Ala His Gln Asn
    130                 135                 140
Glu Asn Pro Ala Val Arg Pro Gln Pro Trp Val Pro Ser Leu Phe Ser
145                 150                 155                 160
Cys Thr Leu Pro Leu Ile Leu Leu Leu Ser Leu Trp
                165                 170

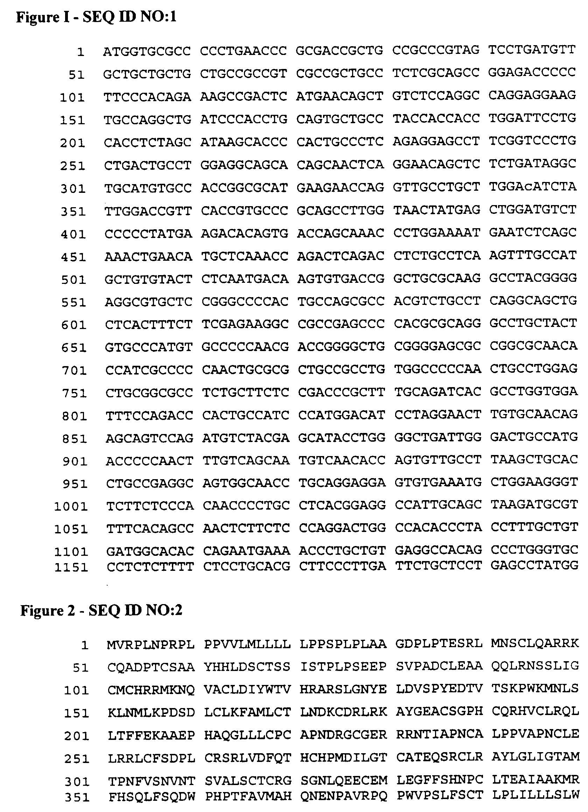

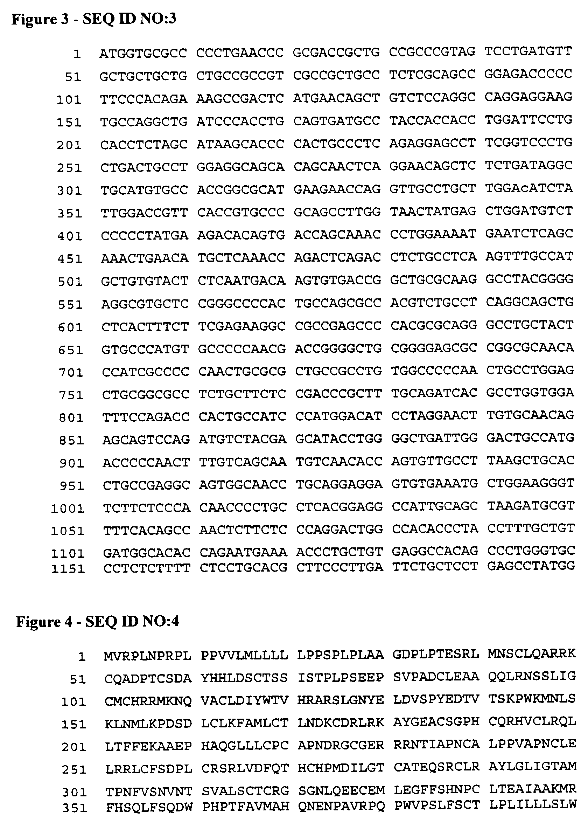

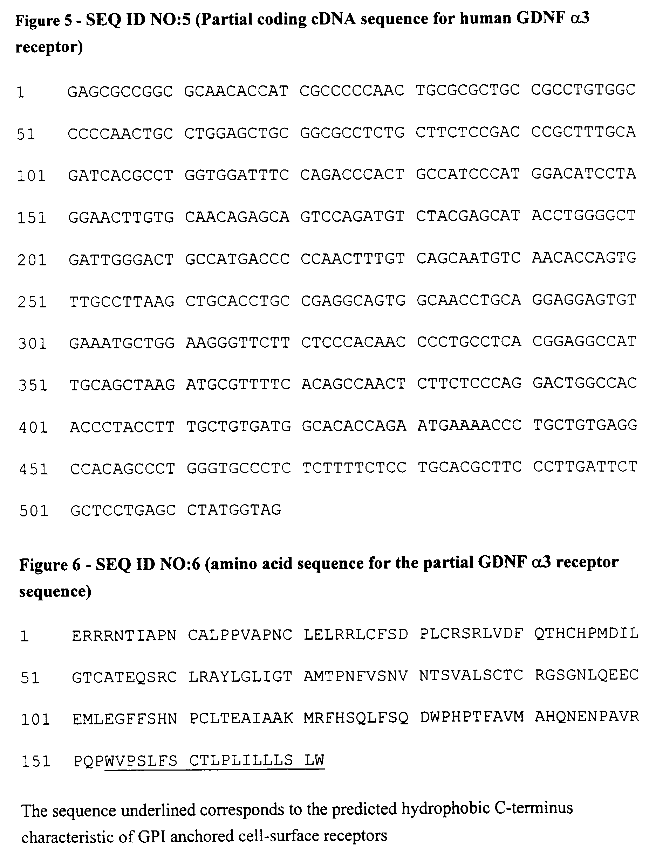

## Claims

1. An isolated polypeptide comprising an amino acid sequence which has at least 80% identity to the amino acid sequence of SEQ ID NO:2 over the entire length of of SEQ ID NO:2.

2. An isolated polypeptide as claimed in claim I in which the amino acid sequence has at least 90%.

3. An isolated polypeptide as claimed in claim 1 in which the amino acid sequence has at least 95%.

4. The polypeptide as claimed in claim 1 which comprises the amino acid sequence of SEQ ID NO:2 or SEQ ID NO:4.

5. The polypeptide of SEQ ID NO:2 or SEQ ID NO:4.

6. An isolated polynucleotide comprising a nucleotide sequence that has at least 80% identity to a nucleotide sequence encoding the polypeptide of SEQ ID NO:2 over the entire coding region; or a nucleotide sequence complementary to said isolated polynucleotide.

7. An isolated polynucleotide as claimed in claim 6 in which the nucleotide sequence has at least 90% identity.

8. An isolated polynucleotide as claimed in claim 6 in which the nucleotide sequence has at least 95% identity.

9. An isolated polynucleotide which comprises the nucleotide sequence contained in SEQ ID NO: 1 encoding the polypeptide of SEQ ID NO:2; or a nucleotide sequence complementary to said isolated polynucleotide.

10. An isolated polynucleotide which comprises a nucleotide sequence which has at least 70% identity to that of SEQ ID NO: 1 over the entire length of SEQ ID NO:1; or a nucleotide sequence complementary to said isolated polynucleotide.

11. An isolated polynucleotide as claimed in claim 10 in which the nucleotide sequence which has at least 90% identity.

12. An isolated polynucleotide as claimed in claim 10 in which the nucleotide sequence which has at least 95% identity.

13. The polynucleotide of claim 10 which is the polynucleotide of SEQ ID NO:1 or SEQ ID NO:3.

14. A DNA or RNA molecule comprising an expression system which is capable of producing a polypeptide comprising an amino acid sequence which has at least 80% identity with the polypeptide of SEQ ID NO:2 when said expression system is present in a compatible host cell.

15. A host cell comprising the expression system of claim 14.

16. A process for producing a polypeptide comprising culturing a host of claim 15 under conditions sufficient for the production of said polypeptide and recovering the polypeptide from the culture.

17. An antibody immunospecific for the polypeptide of claim 1.

18. An agonist or antagonist to the polypeptide of claim 1.

19. An agonist or antagonist to the polypeptide of claim I for use in therapy.

20. An isolated polynucleotide comprising a nucleotide sequence that has at least 80% identity to a nucleotide sequence encoding the polypeptide of SEQ ID NO:2 over the entire length of the encoding region, or a nucleotide sequence complementary to said nucleotide sequence for use in therapy.

21. A nucleic acid molecule that inhibits the expression of the nucleotide sequence encoding polypeptide of claim 1 for use in therapy.

22. A polypeptide that competes with the polypeptide of claim 1 for its ligand, substrate, or receptor, for use in therapy.

23. A process for diagnosing a disease or a susceptibility to a disease in a subject related to expression or activity of the polypeptide of claim 1 in a subject comprising:
(a) determining the presence or absence of a mutation in the nucleotide sequence encoding said polypeptide in the genome of said subject; and/or
(b) analyzing for the presence or amount of said polypeptide expression in a sample derived from said subject

24. A polynucleotide obtainable by screening an appropriate library under stingent hybridization conditions with a labeled probe having the sequence of SEQ ID NO: 1 or a fragment thereof.

25. A GDNF alpha 3 receptor characterised by the deduced amino acid sequence of SEQ ID NO:6; or a fragment thereof

26. A polypeptide which has the amino acid sequence of SEQ ID NO:6

27. A polynucleotide which encodes a polypeptide characterised by the deduced amino acid sequence of SEQ ID NO:6.

28. A polynucleotide comprising the partial DNA sequence given in SEQ ID NO:5.

29. The polynucloetide which has the sequence given in SEQ ID NO:5.
